(19) Europäisches Patentamt
European Patent Office
Office européen des brevets

(11) EP 4 039 194 A1

(12) **EUROPEAN PATENT APPLICATION**
published in accordance with Art. 153(4) EPC

(43) Date of publication:
**10.08.2022 Bulletin 2022/32**

(21) Application number: **20870459.3**

(22) Date of filing: **29.09.2020**

(51) International Patent Classification (IPC):
***A61B 8/00*** *(2006.01)* ***G06T 7/00*** *(2017.01)*
***G06N 20/00*** *(2019.01)* ***A61B 5/055*** *(2006.01)*

(52) Cooperative Patent Classification (CPC):
**A61B 5/055; A61B 8/00; G06N 20/00; G06T 7/00**

(86) International application number:
**PCT/JP2020/036985**

(87) International publication number:
**WO 2021/065937 (08.04.2021 Gazette 2021/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.10.2019 JP 2019181165**

(71) Applicant: **Hitachi High-Tech Corporation**
**Minato-ku**
**Tokyo 105-6409 (JP)**

(72) Inventors:
- **HATTORI Hideharu**
  **Tokyo 100-8280 (JP)**
- **KAKISHITA Yasuki**
  **Tokyo 100-8280 (JP)**
- **UCHIDA Kenko**
  **Tokyo 105-6409 (JP)**
- **ASO Sadamitsu**
  **Tokyo 105-6409 (JP)**
- **SAKURAI Toshinari**
  **Tokyo 105-6409 (JP)**

(74) Representative: **MERH-IP Matias Erny Reichl Hoffmann**
**Patentanwälte PartG mbB**
**Paul-Heyse-Strasse 29**
**80336 München (DE)**

# (54) MACHINE LEARNING APPARATUS

(57) An appropriate learning database and an appropriate classifier are to be created and used. A machine learning device includes: a processor configured to process a data sample; and a storage device configured to store a result of the process. The processor is configured to create a plurality of classifiers based on a plurality of learning databases. Each of the plurality of learning databases stores a plurality of learning data samples. The processor is configured to create an evaluation result on identification performance of each of the plurality of classifiers, and determine, based on the evaluation result, one learning database among the plurality of learning databases and a classifier to be generated based on the one learning database as a learning database and a classifier that are to be used.

[FIG. 1]

1
IMAGE DATA (ex. MOVING IMAGE DATA)
MACHINE LEARNING DEVICE
CONTROL UNIT
91
INPUT UNIT
10
CONNECTED TO EACH ELEMENT
101
LEARNING IMAGE DB (DETERMINED + BEFORE DETERMINATION)
100
LEARNING IMAGE DB (DETERMINED)
LEARNING UNIT
11
102
SUITABILITY EVALUATION UNIT
12
EVALUATION IMAGE
UPDATE DETERMINATION UNIT
13
90
MEMORY
DRAWING UNIT
14
IDENTIFICATION RESULT

EP 4 039 194 A1

## Description

CROSS-REFERENCE TO RELATED APPLICATIONS

The present application claims priority from Japanese Patent Application No. 2019-181165 filed on October 1, 2019, contents of which are incorporated into the present application by reference.

Technical Field

The present invention relates to machine learning, and is applicable to, for example, an image processing technique using machine learning for detecting a specific object (for example, cancer cells or bubbles on a liquid surface) included in a captured image.

Background Art

In recent years, as an image recognition technique, an image recognition technique using machine learning or the like has been studied. By using Deep Learning or the like, detection accuracy of an object in an image is improved. In order to develop a classifier that detects the object in the image, for example, a technique proposed in PTL 1 is provided. In PTL 1, a plurality of learning image data groups are set to execute machine learning, and parameters of a neural network are calculated.

Citation list

Patent Literature

PTL 1: JP-A-2016-143351

Summary of Invention

Technical Problem

However, as in PTL 1, even when learning images are divided into a plurality of image groups and relearned to obtain parameters, the image groups may include an image that does not contribute to an improvement in identification accuracy of a classifier, and the identification accuracy of the classifier is not necessarily improved. In PTL 1, it is impossible to create a learning image database capable of continuously improving the identification accuracy of the classifier.

Solution to Problem

A machine learning device according to an aspect of the invention includes: a processor configured to process a data sample; and a storage device configured to store a result of the process. The processor is configured to: create a plurality of classifiers based on a plurality of learning databases, each of the plurality of learning databases storing a plurality of learning data samples; create an evaluation result on identification performance of each of the plurality of classifiers; and determine, based on the evaluation result, one learning database among the plurality of learning databases and a classifier to be generated based on the one learning database as a learning database and a classifier that are to be used.

Further features related to the invention are clarified based on the description of the present specification and accompanying drawings. Aspects of the invention are achieved and implemented utilizing elements, combinations of various elements, the following detailed description, and accompanying claims. It should be understood that the description of the present specification is merely exemplary, and is not intended to limit the scope of claims or application examples of the invention in any sense.

Advantageous Effect

According to an aspect of the invention, an appropriate learning database and an appropriate classifier can be created and used.

Brief Description of Drawings

[FIG. 1] FIG. 1 is a block diagram illustrating functions of a machine learning device according to a first embodiment.
[FIG. 2A] FIG. 2A is a diagram illustrating a configuration example of hardware of the machine learning device according to the first embodiment.
[FIG. 2B] FIG. 2B is a diagram illustrating a configuration example of a learning unit according to the first embodiment.
[FIG. 3] FIG. 3 is a diagram illustrating an example of an operation of the learning unit according to the first embodiment.
[FIG. 4] FIG. 4 is a diagram illustrating an example of the operation of the learning unit according to the first embodiment.
[FIG. 5] FIG. 5 is a diagram illustrating an example of the operation of the learning unit according to the first embodiment.
[FIG. 6] FIG. 6 is a diagram illustrating an example of the operation of the learning unit according to the first embodiment.
[FIG. 7] FIG. 7 is a diagram illustrating an example of the operation of the learning unit according to the first embodiment.
[FIG. 8] FIG. 8 is a diagram illustrating an example of an operation of a drawing unit according to the first embodiment.
[FIG. 9] FIG. 9 is a flowchart illustrating the operation of the learning unit according to the first embodiment.
[FIG. 10] FIG. 10 is a flowchart illustrating an overall operation of the machine learning device according to the first embodiment.
[FIG. 11] FIG. 11 is a diagram illustrating an example of an update status display of the drawing unit according to the first embodiment.
[FIG. 12] FIG. 12 is a block diagram illustrating functions of a machine learning device according to a second embodiment.
[FIG. 13] FIG. 13 is a flowchart illustrating an overall operation of the machine learning device according to the second embodiment.
[FIG. 14] FIG. 14 is a diagram illustrating a schematic configuration of a remote diagnosis support system on which an image diagnosis support device including a machine learning device according to a third embodiment is mounted.
[FIG. 15] FIG. 15 is a diagram illustrating a schematic configuration of a network contract service providing system on which an image diagnosis support device including a machine learning device according to a fourth embodiment is mounted.

Description of Embodiments

In an embodiment, machine learning is executed using images in learning image databases to create a plurality of classifiers, evaluation results are further obtained by evaluating the plurality of created classifiers, and whether the classifiers and the learning image databases can be updated is controlled by determining a plurality of evaluation results. Accordingly, a machine learning device and a machine learning method are provided for implementing creations of a classifier capable of identifying an object (for example, a tissue or a cell) in an image with high accuracy and a learning image database including an image contributing to a continuous improvement in identification accuracy of the classifier.

Hereinafter, embodiments of the invention will be described with reference to the accompanying drawings. In the accompanying drawings, functionally the same element may be displayed with the same number. The accompanying drawings show specific embodiments and implementation examples according to the principle in the invention. The accompanying drawings, the embodiments, and the implementation examples are for understanding the invention and are not used to limit the invention.

The present embodiment is described in sufficient detail in order for a person skilled in the art to implement the invention, but it should be understood that other implementations and aspects are possible, and changes in configuration and structure and replacement of various elements are possible without departing from the scope and spirit of the technical idea of the invention. Therefore, the following description should not be construed as being limited to the present embodiment.

Further, as will be described later, the embodiments of the invention may be implemented by software running on a general purpose computer, or may be implemented by dedicated hardware or a combination of software and hardware. Hereinafter, each process in the embodiments of the invention will be described with "each processing unit (for example, a learning unit) serving as a program" as a subject (operation subject). Since a program is executed by a processor (CPU or the like) to execute a determined process using a memory and a communication port (communication control device), the description may be made with the processor as the subject.

(1) First Embodiment

<Functional Configuration of Machine Learning Device>

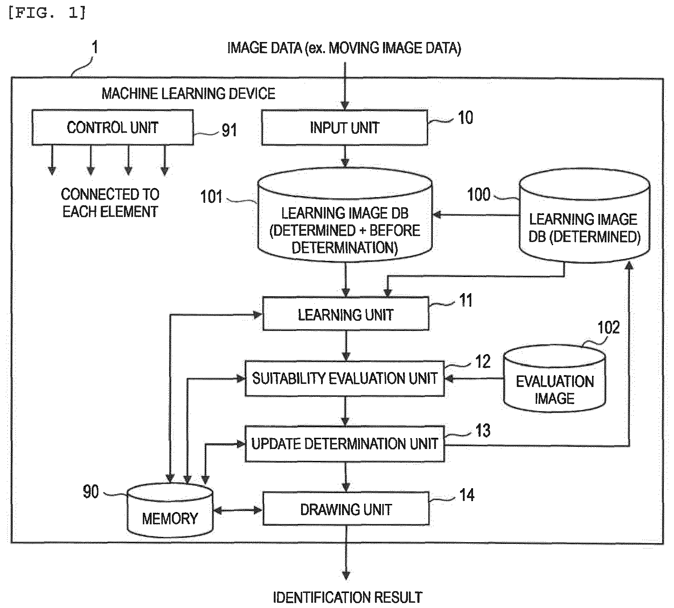

FIG. 1 is a block diagram illustrating a functional configuration of a machine learning device according to a first embodiment. A machine learning device 1 includes an input unit 10, a learning unit 11, a suitability evaluation unit 12, an update determination unit 13, a drawing unit 14, a control unit 91, a learning image database (DB) (determined) 100, a learning image DB (determined + before determination) 101, an evaluation image 102, and a memory 90. The machine learning device 1 may be implemented in an image acquisition device such as an image diagnosis support device, or may be implemented in, as will be described later (third to fourth embodiments), a server to be connected to the image acquisition device via a network.

The input unit 10, the learning unit 11, the suitability evaluation unit 12, the update determination unit 13, and the drawing unit 14 in the machine learning device 1 may be implemented by a program or a processor that executes the program, or may be implemented by a hardware module.

Image data is input to the input unit 10. For example, the input unit 10 may acquire, as an input image, encoded still image data in a format of JPG, Jpeg2000, PNG, BMP, or the like captured at predetermined time intervals by an image capturing unit such as a camera built in the image acquisition device (not illustrated in FIG. 1).

The input unit 10 may extract the still image data of frames at predetermined intervals from moving image data in a format of MotionJPEG, MPEG, H.264, HD/SDI, or the like, and acquire an image of the still image data as an input image. The input unit 10 may acquire the input image from an image capturing unit via a bus, a network, or the like. The input unit 10 may acquire an image already stored in a detachable recording medium as an input image. The image input by the input unit 10 is stored in the learning image DB (determined + before determination) 101 as a learning image (before determination).

The learning image DB (determined) 100 stores a plurality of combinations of images determined as learning images and correct labels. Correct labels corresponding to the images are determined. The learning image (before determination) includes a plurality of combinations of images before being determined as the learning images and the correct labels. The correct labels corresponding to the images may be changed.

The learning unit 11 executes the machine learning such that an image of a specific object in the input image is identified as the specific object, for example, an image of a normal tissue or cell is identified as the normal tissue or cell, and an image of an abnormal tissue or cell in the input image is identified as the abnormal tissue or cell. The learning unit 11 creates a classifier CA (parameters (a filter coefficient, an offset value, and the like) necessary for identification) based on the learning image DB (determined) 100. The learning unit 11 creates a classifier CB based on the learning image DB (determined + before determination) 101 obtained by adding the learning image (before determination) input by the input unit 10 to the learning image DB (determined) 100.

The suitability evaluation unit 12 calculates identification results and identification values of the classifiers CA and CB using the evaluation image 102. The update determination unit 13 controls whether the classifier CA and the learning image DB (determined) 100 can be updated using the identification results and the identification values of the classifiers CA and CB obtained by the suitability evaluation unit 12. The update determination unit 13 stores, in the memory 90, information such as later-described $Ave_{dr1}$ and $Ave_{dr2}$ that are obtained by the suitability evaluation unit 12, each $M_{drN}$, the number of updates of the classifier CA and the learning image DB (determined) determined by the update determination unit 13, and transition of the updated $Ave_{dr1}$.

The drawing unit 14 outputs, to an output device such as a display or a printer, the information such as the later-described $Ave_{dr1}$ and $Ave_{dr2}$ that are obtained by the suitability evaluation unit 12, each $M_{drN}$, the number of updates of the classifier CA and the learning image DB (determined) determined by the update determination unit 13, and transition of the updated $Ave_{dr1}$.

The control unit 91 is implemented by, for example, a processor that executes a program, and is connected to each element in the machine learning device 1. Each component of the machine learning device 1 operates as described above autonomously or in response to an instruction from the control unit 91.

As described above, in the machine learning device 1 according to the present embodiment, the learning unit 11 executes the machine learning to create the classifier CA from the learning image DB (determined) 100, and to create the classifier CB based on the learning image DB (determined + before determination) 101 obtained by adding the learning image (before determination) input by the input unit 10 to the learning image DB (determined) 100. The suitability evaluation unit 12 calculates the identification results and the identification values of the classifiers CA and CB using the evaluation image 102. The update determination unit 13 controls whether the classifier CA and the learning image DB (determined) 100 can be updated using the identification results and the identification values of the classifiers CA and CB obtained by the suitability evaluation unit 12.

<Hardware Configuration of Machine Learning Device>

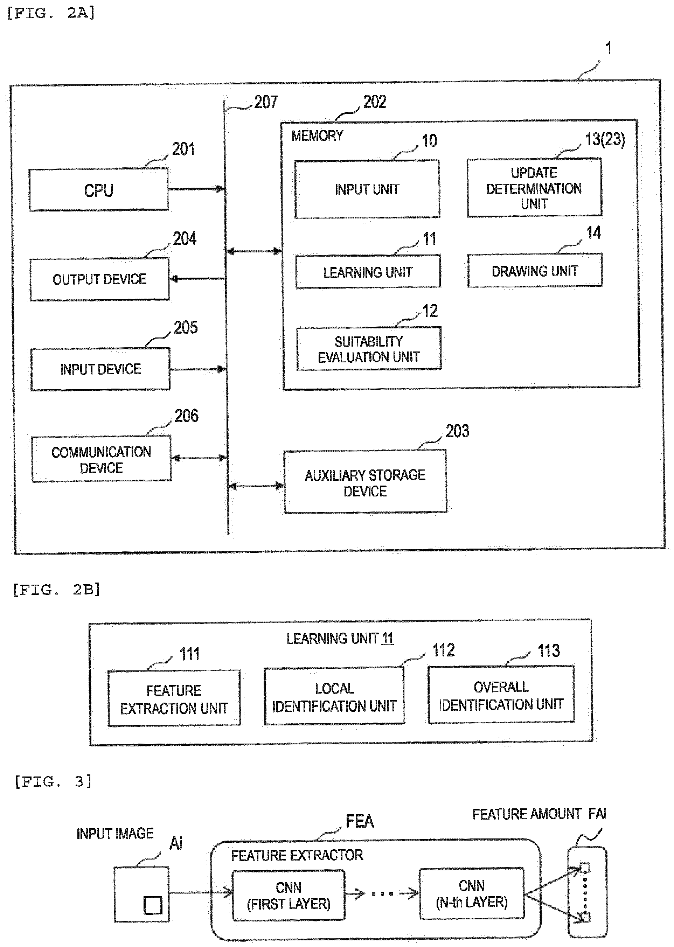

FIG. 2A is a diagram illustrating a configuration example of hardware of the machine learning device 1 according to the first embodiment. The machine learning device 1 includes a CPU (processor) 201 that executes various programs, a memory 202 (main storage device) that stores various programs, and an auxiliary storage device 203 (equivalent to the memory 90) that stores various types of data. The machine learning device 1 further includes an output device 204 that outputs an identification result and an update possibility result of the classifier and the learning image DB (determined) 100, an input device 205 that inputs an instruction of a user, an image, or the like, and a communication device 206 that executes communication with another device. These components in the machine learning device 1 are connected to one another by a bus 207.

The CPU 201 reads various programs from the memory 202 as necessary and executes the programs. The memory 202 stores the input unit 10, the learning unit 11, the suitability evaluation unit 12, the update determination unit 13, and the drawing unit 14 as programs.

The auxiliary storage device 203 stores the learning image (before determination), parameters of the classifiers CA and CB generated by the learning unit 11, the identification results and the identification values generated by the suitability evaluation unit 12, and an update result determined by the update determination unit 13. The auxiliary storage device 203 further stores the learning image DB (determined) 100, the learning image DB (determined + before determination) 101, position information for drawing a detection frame generated by the drawing unit 14, and the like. Each of the memory 202, the auxiliary storage device 203, and a combination thereof is a storage device.

The output device 204 includes devices such as a display, a printer, and a speaker. For example, the output device 204 is a display device, and displays data generated by the drawing unit 14 on a screen. The input device 205 includes devices such as a keyboard, a mouse, and a microphone. The instruction (including determination of a learning image (before determination) input) of the user is input to the machine learning device 1 by the input device 205.

The communication device 206 is not essential in the machine learning device 1, and in a case in which a communication device is provided in a personal computer or the like connected to the image acquisition device, the machine learning device 1 may not hold the communication device 206. The communication device 206 receives, for example, data (including an image) transmitted from another device (for example, a server) to be connected via a network, and stores the data in the auxiliary storage device 203.

The machine learning device according to the present embodiment executes the machine learning using the images in the learning image databases to create a plurality of classifiers, and further evaluates the plurality of created classifiers to obtain evaluation results. The machine learning device can create the classifier capable of identifying the object (for example, a tissue or a cell) in the image with higher accuracy and the learning image database including the image contributing to a continuous improvement in identification accuracy of the classifier by determining the evaluation results and controlling whether the classifier and the learning image database can be updated.

<Configuration and Operation of Each Unit>

Hereinafter, the configuration and the operation of each element will be described in detail.

(i) Learning Unit 11

FIG. 2B illustrates a configuration example of the learning unit 11. The learning unit 11 includes a feature extraction unit 111, a local identification unit 112, and an overall identification unit 113.

(i-i) Feature Extraction Unit 111

The feature extraction unit 111 obtains a feature amount of the input image. FIG. 3 illustrates an example of obtaining the feature amount. CNN in FIG. 3 represents a convolutional neural network. For example, the feature extraction unit 111 obtains a feature amount FAi of an object (for example, a tissue or a cell) of an input image Ai from the input image Ai using a feature extractor FEA that executes a calculation according to Equation 1.
[Math 1]

$$fi = h\left(\sum_{j=1}^{m}(pj \times wj) + bi\right) \quad \text{Equation 1}$$

The learning unit 11 obtains a filter coefficient wj by the machine learning such that an image of each object is identified as each object (a normal tissue or a normal cell is identified as a normal tissue or a normal cell, or an abnormal tissue or an abnormal cell is identified as an abnormal tissue or an abnormal cell). Here, pj represents a pixel value, bi represents an offset value, m represents the number of filter coefficients, and h represents a nonlinear function.

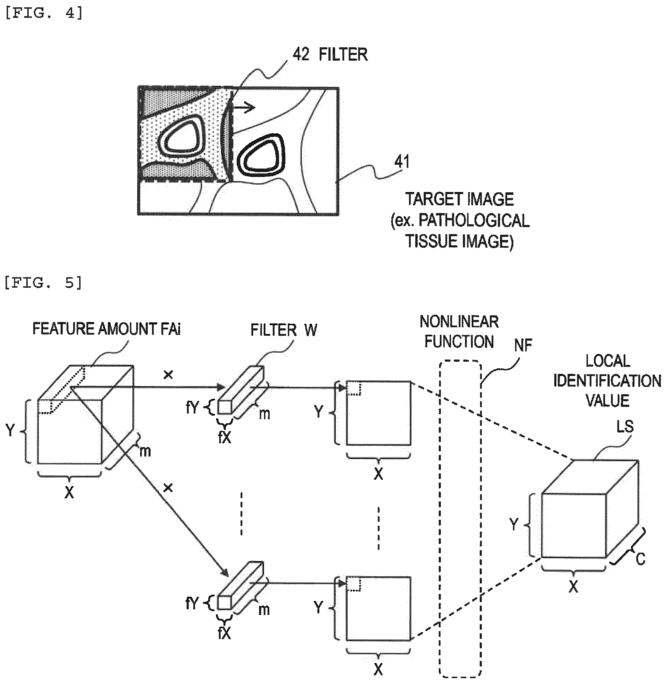

As illustrated in FIG. 4, the feature extraction unit 111 obtains a feature amount fi of any filter i by obtaining a calculation result of each filter 42 from the upper left to the lower right of a target image (for example, a pathological tissue image) 41 according to Equation 1. For example, a matrix of feature amounts fi obtained by the feature extractor FEA is set as the feature amount FAi of the input image Ai. A method for creating the feature extractor FEA will be described later.

(i-ii) Local Identification Unit 112

As illustrated in FIG. 5, the local identification unit 112 calculates a value of an object likelihood (for example, a lesion likelihood) for each local region according to Equation 2 using the feature amount FAi of the feature extractor FEA obtained by the feature extraction unit 111 and a nonlinear function NF (for example, a sigmoid function). The local identification unit 112 determines, based on the calculated value, whether an object in the input image Ai is an object (for example, a normal cell or an abnormal cell) to be detected.
[Math 2]

$$LS(c,y,x) = sigmoid\left(\sum_{j=0}^{m}\sum_{fy=0}^{fY}\sum_{fx=0}^{fX} FAi(j, y+fy, x+fx) \times W(c,j,fy,fx) + B(c)\right)$$

Equation 2

In Equation 2, LS represents a local identification value including a three-dimensional array of a class, a height, and a width, and FAi represents a feature amount including a three-dimensional array of a feature amount number, a height, and a width that are obtained by the feature extraction unit 111. W represents a filter for calculating a local identification value including a four-dimensional array of a class, a feature amount number, a height, and a width, and B represents an offset value for calculating a local identification value including a one-dimensional array of a class. c represents an index of the class, y represents an index of the feature amount in a vertical direction, x represents an index of the feature amount in a horizontal direction, fy represents an index of the filter in the vertical direction, fx represents an index of the filter in the horizontal direction, and j represents an index of the filter.

In Equation 2, the local identification value is calculated using a Convolution process, but the method for calculating the local identification value is not limited thereto. For example, the local identification value may be obtained by applying the Convolution process, the nonlinear function, or the like a plurality of times, or the local identification value may be calculated by inputting a feature amount at each coordinate to another identification method such as Random forest or SVM.

(i-iii) Overall Identification Unit 113

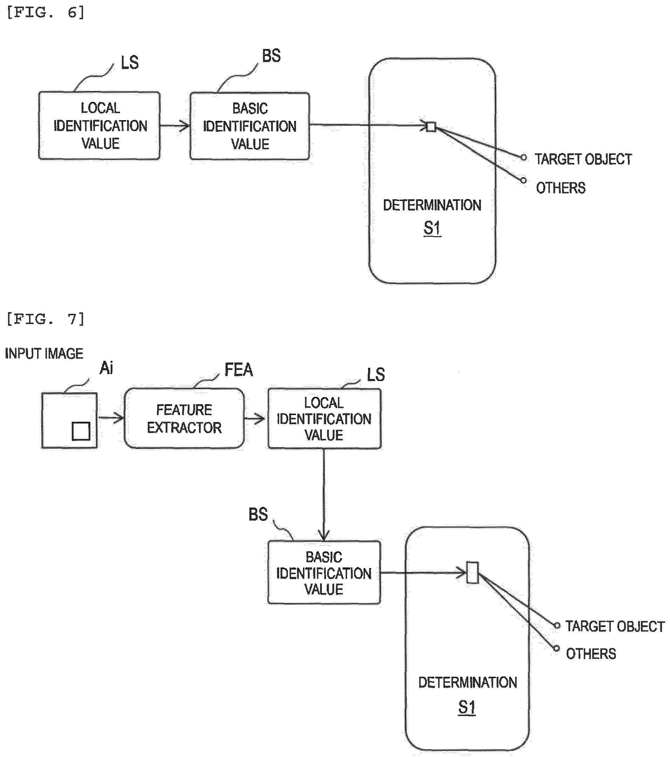

As illustrated in FIG. 6, the overall identification unit 113 obtains a basic identification value BS using a local identification value LS obtained by the local identification unit 112 and the nonlinear function (for example, a sigmoid function). The overall identification unit 113 determines whether the object in the input image Ai is an object (for example, a normal cell or an abnormal cell) to be detected using the basic identification value BS as a calculation result R indicating a value of the object likelihood (for example, the lesion likelihood) of each object image in the input image (S1).

The basic identification value BS is calculated using a global identification value GS in Equation 3 and according to Equation 4.
[Math 3]

$$GS(c) = sigmoid\left(\frac{1}{X*Y}\sum_{y=0}^{Y}\sum_{x=0}^{X}\sum_{j=0}^{m}\sum_{fy=0}^{fY}\sum_{fx=0}^{fX} FAi(j, y+fy, x+fx) \times W(c, j, fy, fx) + B(c)\right)$$

Equation 3

[Math 4]

$$BS = \frac{1}{C}\sum_{c=0}^{C} NLL(GS(c), Label(c))$$

Equation 4

In Equation 3, GS represents a global identification value including a one-dimensional array of a class, FAi represents the feature amount including the three-dimensional array of the feature amount number, the height, and the width that are obtained by the feature extraction unit 111, and W represents a filter for calculating a global identification value including a four-dimensional array of a class, a feature amount number, a height, and a width. B represents an offset value for calculating a global identification value including a one-dimensional array of a class, and c represents an index of the class. y represents the index of the feature amount in the vertical direction, x represents the index of the feature amount in the horizontal direction, fy represents the index of the filter in the vertical direction, fx represents the index of the filter in the horizontal direction, and j represents the index of the filter.

Label in Equation 4 indicates a teacher label (correct label) in an image unit including a one-dimensional array of a class. The learning unit 11 to be described later obtains the coefficient of the filter W and the offset value B that are to be updated in Equation 3 by the machine learning. NLL represents a loss function, for example, a negative log likelihood.

In Equation 3, the global identification value is calculated using the Convolution process and an average process in the horizontal and vertical directions, but the method for calculating the global identification value is not limited thereto. For example, the average process in the horizontal and vertical directions may be executed after the Convolution process, the nonlinear function, or the like is applied a plurality of times, or average values in the horizontal and vertical directions of values obtained by inputting the feature amount at each coordinate to another identification method such as Random forest or SVM may be calculated. The process is not limited to the average process in the horizontal and vertical directions, and may be a summation process or the like.

The learning unit 11 learns the feature amount of each object using a present machine learning technique such that the overall identification unit 113 identifies each object in the input image as each object (for example, a normal tissue or a normal cell is identified as a normal tissue or a normal cell, or an abnormal tissue or an abnormal cell is identified as an abnormal tissue or an abnormal cell) using the local identification value, and obtains the coefficient of the filter W and the offset value B. For example, the convolutional neural network may be used as the machine learning technique.

As illustrated in FIG. 7, by executing the machine learning in advance, the learning unit 11 calculates the feature amount FAi of the input image Ai according to Equation 1 using the input image Ai (for example, a pathological image). Next, the learning unit 11 obtains the local identification value LS based on the feature amount FAi according to Equation 2. The learning unit 11 calculates the parameters in Equations 1 to 3 using the basic identification value BS obtained based on the local identification value LS, such that the image of the target object is determined as the target object (for example, an abnormal tissue or an abnormal cell is determined as an abnormal tissue or an abnormal cell, and a normal tissue or a normal cell is determined as a normal tissue or a normal cell).

The learning unit 11 repeatedly executes processes of the feature extraction unit 111, the local identification unit 112, and the overall identification unit 113 using a plurality of learning images of the learning image DB (determined) 100, and obtains the parameters (the filter coefficient wj, the coefficient of the filter W, the offset values bi and B, and the like) shown in Equations 1, 2, and 3.

The learning unit 11 creates the classifier CA including the feature extractor that calculates the feature amount of the input image based on the input image, a local classifier that obtains the local identification value, and a global classifier that obtains the global identification value. Similarly, the learning unit 11 obtains the parameters (the filter coefficient wj, the coefficient of the filter W, the offset values bi and B, and the like) shown in Equations 1, 2, and 3 using a plurality of learning images in the learning image DB (determined + before determination) 101, and creates the classifier CB. The learning unit 11 stores the obtained parameters (the filter coefficient wj, the coefficient of the filter W, the offset values bi and B, and the like) in the memory 90.

For example, the learning unit 11 creates the classifiers CA and CB by adjusting a balance between the numbers of images of identification types of the learning image DB (determined) 100 and the learning image DB (determined + before determination) 101. For example, the learning unit 11 adjusts the number of images such that a difference in the number of images between the identification types of the learning image DB (determined) 100 and the learning image DB (determined + before determination) 101 is smaller than a predetermined threshold. Accordingly, more appropriate evaluations can be executed.

(ii) Suitability Evaluation Unit 12

For the classifiers CA and CB created by the learning unit 11, the suitability evaluation unit 12 evaluates the classifiers by obtaining $Ave_{drX}$ ($Ave_{dr1}$) of the classifier CA and $Ave_{drX}$ ($Ave_{dr2}$) of the classifier CB according to Equation 5 using the evaluation image 102. In Equation 5, N represents the number of types to be identified. Each $M_{drN}$ indicates a target detection rate. For example, if N = 2, $M_{dr1}$ represents a benign tumor detection rate (average), and $M_{dr2}$ represents a malignant tumor detection rate (average). The suitability evaluation unit 12 can use a value different from $Ave_{drX}$, and may use, for example, the maximum value of $M_{drN}$.
[Math 5]

$$Ave_{drX} = (M_{dr1} + \cdots + M_{drN})/N \qquad \text{Equation 5}$$

(iii) Update Determination Unit 13

The update determination unit 13 controls whether the classifier CA and the learning image DB (determined) 100 can be updated by comparing a plurality of $Ave_{drX}$ obtained by the suitability evaluation unit 12. For example, in a case in which $Ave_{dr2} > Ave_{dr1}$ and $Ave_{dr2} > TH1$ (for example, TH1 = 0.7) (K1), the update determination unit 13 updates the classifier CA and the learning image DB (determined) 100. Specifically, the update determination unit 13 updates a content of the learning image DB (determined) 100 to a content of the learning image DB (determined + before determination) 101, and updates a content of the classifier CA to a content of the classifier CB learned in the learning image DB (determined + before determination) 101.

In a case in which $Ave_{dr2} \leq Ave_{dr1}$ and $Ave_{dr2} > TH1$ (K2), the update determination unit 13 does not update the classifier CA and the learning image DB (determined), and changes an order of images in the learning image DB (before determination), for example, at random. The learning unit 11 executes relearning using the learning image DB (before determination) whose order of images is changed. In a case of neither K1 nor K2, for relearning, the update determination unit 13 changes correct labels of several images in the learning image DB (before determination) automatically or according to an instruction of the user via the input device. For example, the update determination unit 13 may determine whether there is a change in the correct label for each mini batch of the images.

The update determination unit 13 records, in the memory 90 or a log file, $Ave_{dr1}$ and $Ave_{dr2}$ that are obtained by the suitability evaluation unit 12, each $M_{drN}$, the number of updates of the classifier CA and the learning image DB (determined) 100 that are determined by the update determination unit 13, transition of the updated $Ave_{dr1}$, and the like.

(iv) Drawing Unit 14

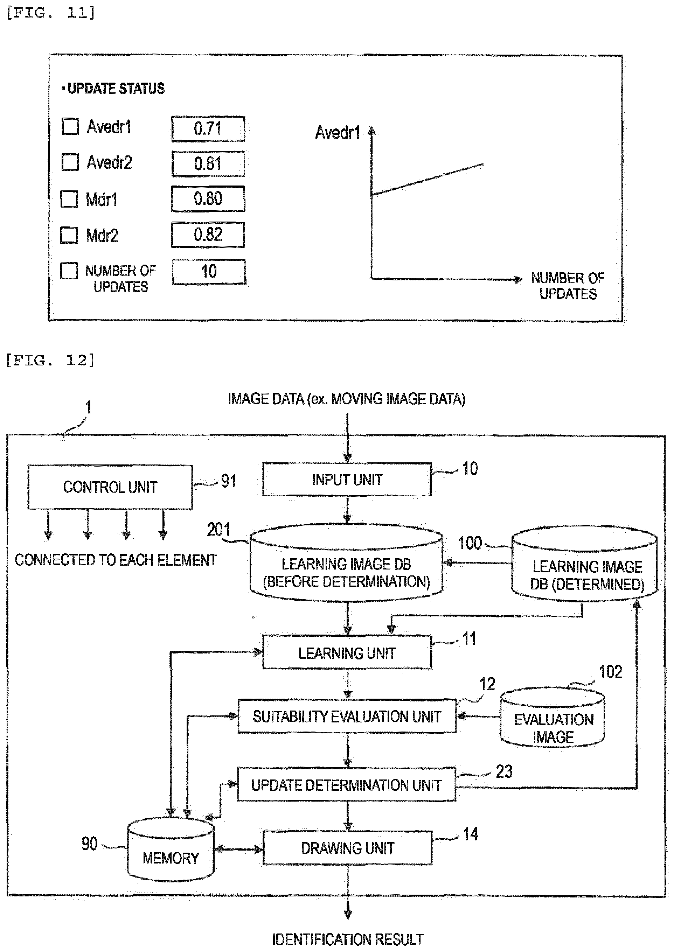

As an example, the drawing unit 14 displays, on a graphical user interface (GUI) illustrated in FIG. 11, $Ave_{dr1}$ and $Ave_{dr2}$ that are obtained by the suitability evaluation unit 12, each $M_{drN}$, the number of updates of the classifier CA and the learning image DB (determined) that are determined by the update determination unit 13, the transition of the updated $Ave_{dr1}$, and the like. In the example in FIG. 11, $Ave_{dr1}$ is displayed as 0.71, $Ave_{dr2}$ is displayed as 0.81, $M_{dr1}$ is displayed as 0.80, $M_{dr2}$ is displayed as 0.82, and the number of updates is displayed as 10.

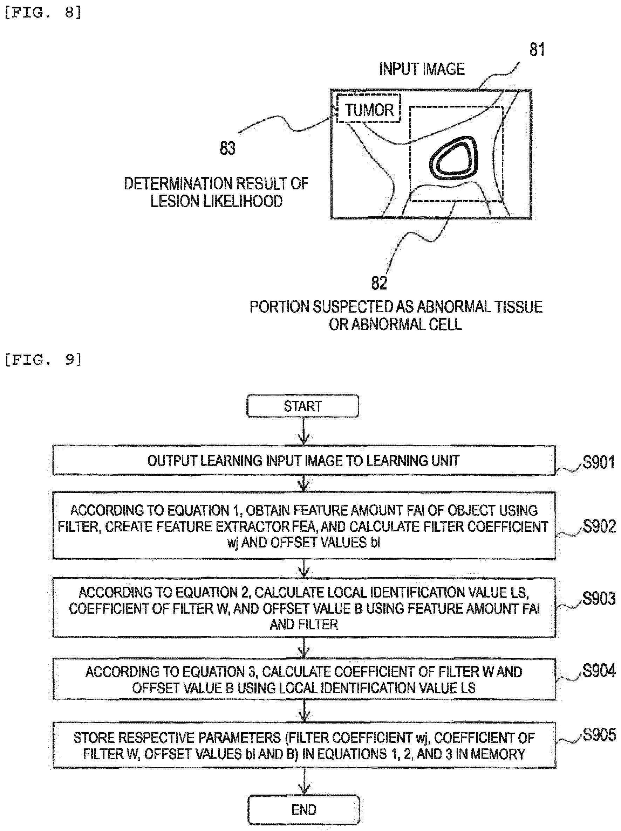

The drawing unit 14 displays an identification result obtained by each classifier for an unknown image input by the input unit 10. In a case in which a specific portion in the image is determined as an object (for example, an abnormal tissue or an abnormal cell) to be detected, as illustrated in FIG. 8, the drawing unit 14 may draw a detection frame 82 in an input target image 81 in order to indicate a portion (for example, a portion suspected as an abnormal tissue or an abnormal cell) of the object to be detected. On the other hand, in a case in which the target image 81 is determined as a normal tissue or a normal cell, the drawing unit 14 may display the input target image 81 as it is without drawing the detection frame 82 on the input target image 81.

As illustrated in FIG. 8, the drawing unit 14 displays a result (for example, a tumor) 83 of the determined object likelihood. The drawing unit 14 is not an essential component of the machine learning device 1, and when the image diagnosis support device is provided with a drawing unit, the machine learning device 1 may not hold the drawing unit 14.

<Processing Procedure of Machine Learning Device>

FIG. 9 is a flowchart illustrating an operation of the learning unit 11 of the machine learning device 1 according to the first embodiment. Hereinafter, the learning unit 11 is described as an operation subject. It may be interpreted that the CPU 201 is the operation subject, and the CPU 201 executes each processing unit as a program.

(i) Step 901

The input unit 10 receives the learning input image Ai and outputs the input image Ai to the learning unit 11.

(ii) Step 902

By executing the machine learning and according to the above-mentioned Equation 1, the learning unit 11 obtains a feature amount of an object (for example, a tissue or a cell) in the input image Ai using a filter, and creates the feature extractor FEA. The learning unit 11 obtains the filter coefficient wj and the offset value bi for the feature amount FAi.

(iii) Step 903

By executing the machine learning and according to Equation 2, the learning unit 11 obtains the local identification value LS based on the feature amount FAi, calculates a value of the object likelihood (for example, a lesion likelihood) for each local region, and obtains the parameters (the coefficient of the filter W, the offset value B, or the like) in Equation 2 for obtaining the local identification value so as to determine whether the object in the input image Ai is an object (for example, a normal cell or an abnormal cell) to be detected.

(iv) Step 904

By executing the machine learning and using the basic identification value BS obtained based on the local identification value LS, the learning unit 11 obtains the parameters (the coefficient of the filter W, the offset value B, and the like) in Equation 3 so as to determine the image of the target object as the target object (for example, an abnormal tissue or an abnormal cell is determined as an abnormal tissue or an abnormal cell, and a normal tissue or a normal cell is determined as a normal tissue or a normal cell).

(v) Step 905

The learning unit 11 stores the parameters (the filter coefficient wj, the coefficient of the filter W, the offset values bi and B, and the like) in Equations 1, 2, and 3 in the memory 90.

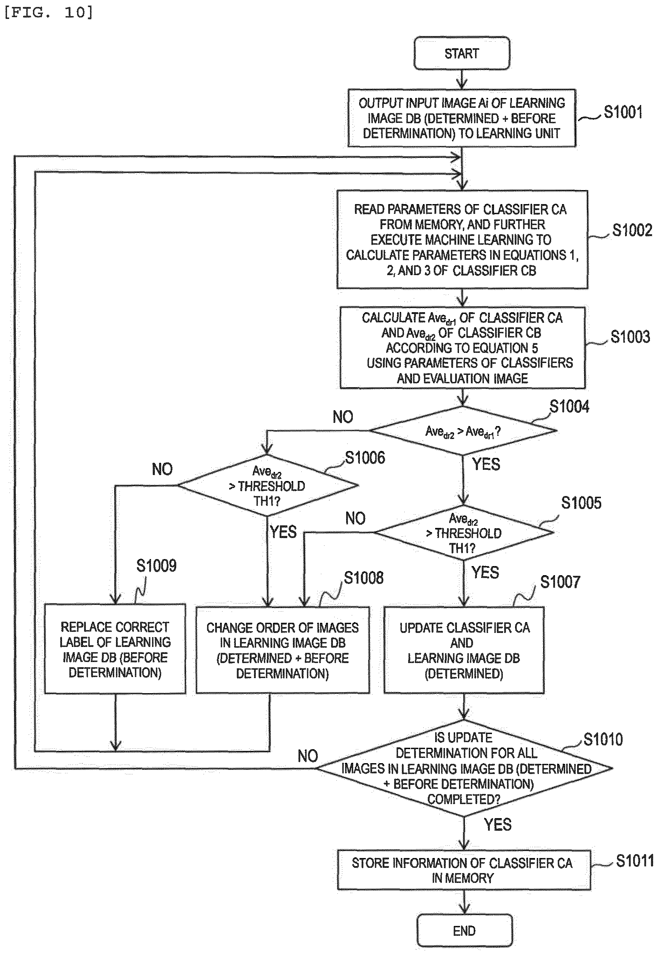

FIG. 10 is a flowchart illustrating an operation of the machine learning device 1 according to the present embodiment. Hereinafter, each processing unit (the input unit 10, the learning unit 11, and the like) is described as an operation subject. It may be interpreted that the CPU 201 is the operation subject, and the CPU 201 executes each processing unit as a program.

(i) Step 1001

The input unit 10 outputs the input image Ai of the learning image DB (determined + before determination) to the learning unit 11.

(ii) Step 1002

The learning unit 11 reads, from the memory 90, the parameters in Equations 1, 2, and 3 related to the classifier CA. The learning unit 11 further performs the machine learning using the learning image DB (determined + before determination) 101, and calculates the respective parameters in Equations 1, 2, and 3 related to the classifier CB.

(iii) Step 1003

The suitability evaluation unit 12 calculates $Ave_{dr1}$ of the classifier CA and $Ave_{dr2}$ of the classifier CB according to Equation 5 using the parameters of the classifiers CA and CB and the evaluation image.

(iv) Step 1004

The update determination unit 13 compares the calculated $Ave_{dr1}$ and $Ave_{dr2}$. In a case in which $Ave_{dr2} > Ave_{dr1}$, the update determination unit 13 proceeds the process to step 1005. On the other hand, in a case in which the calculation result $Ave_{dr2} \leq Ave_{dr1}$, the update determination unit 13 proceeds the process to step 1006.

(v) Step 1005

In a case in which $Ave_{dr2} > TH1$, the update determination unit 13 proceeds the process to step 1007. On the other hand, in a case in which $Ave_{dr2} \leq TH1$, the update determination unit 13 proceeds the process to step 1008.

(vi) Step 1006

In a case in which $Ave_{dr2} > TH1$, the update determination unit 13 proceeds the process to step 1008. On the other hand, in a case in which $Ave_{dr2} \leq TH1$, the update determination unit 13 proceeds the process to step 1009.

(vii) Step 1007

The update determination unit 13 updates the classifier CA and the learning image DB (determined) 100.

(viii) Step 1008

The update determination unit 13 changes the order of the images in the learning image DB (determined + before determination).

(ix) Step 1009

The update determination unit 13 replaces the correct label of the learning image DB (before determination).

(x) Step 1010

The update determination unit 13 checks whether an update determination for all the images in the learning image DB (determined + before determination) is completed, and proceeds the process to step 1011 if the update determination is completed. On the other hand, if the update determination is not completed, the update determination unit 13 returns the process to step 1002 and repeats step 1002 to step 1009.

(xi) Step 1011

The update determination unit 13 stores information of the classifier CA in the memory 90 (equivalent to the auxiliary storage device 203).

As described above, even in a case in which the learning image database includes an image that does not contribute to the improvement in identification accuracy of the classifier, the machine learning device automatically determines an image that contributes to the improvement in identification accuracy of the classifier, and controls whether the classifier and the learning image database can be updated according to a determination result. More specifically, the machine learning device executes the machine learning using images in a plurality of learning image databases to create a plurality of classifiers, and further evaluates the plurality of created classifiers to obtain evaluation results. The machine learning device determines a learning image database and a classifier that are to be used by determining a plurality of evaluation results and controlling whether the classifiers and the learning image databases can be updated. Accordingly, it is possible to create the classifier capable of identifying the object (for example, a tissue or a cell) in the image with high accuracy and the learning image database including the image contributing to a continuous improvement in identification accuracy of the classifier.

Even in a case in which learning images to be input includes images that do not contribute to the improvement in identification accuracy of the classifier, it is possible to create the learning image database by excluding these images. Even in a case in which the input learning images do not contribute to the improvement in identification accuracy of the classifier at that time, it is possible to use the learning images as images contributing to the improvement in identification accuracy of the classifier by changing the order of the images to be learned and relearning the images.

(2) Second Embodiment

Hereinafter, the second embodiment will be described. The machine learning device 1 according to a second embodiment illustrated in FIG. 12 includes many components similar to those according to the first embodiment illustrated in FIG. 1, includes a learning image DB (before determination) 201 instead of the learning image DB (determined + before determination) 101 according to the first embodiment, and includes an update determination unit 23. Hereinafter, configurations different from those in FIG. 1 will be mainly described.

The machine learning device 1 according to the present embodiment executes the machine learning using images in the learning image databases to create a plurality of classifiers, and further evaluates the plurality of created classifiers to obtain evaluation results. The machine learning device 1 determines a plurality of evaluation results and controls whether the classifier and the learning image database can be updated or creation of the classifier and the learning image database. Accordingly, it is possible to create, for example, the classifier capable of identifying the object (for example, a tissue or a cell) in the image with high accuracy and the learning image database including an image contributing to a continuous improvement in identification accuracy of the classifier. The classifier and the learning image database are suitable for each facility or each period.

<Configuration and Operation of Each Unit>

Hereinafter, the configuration and the operation of each element that are different from those in FIG. 1 will be described in detail.

(i) Learning Image DB (before determination) 201

The learning image DB (before determination) 201 stores an image input by the input unit 10, and does not store other images.

(ii) Update Determination Unit 23

The learning unit 11 creates the classifier CA based on the learning image DB (determined) 100, and creates the classifier CB based on the learning image DB (before determination) 201. The suitability evaluation unit 12 evaluates the classifiers by obtaining $Ave_{dr1}$ of the classifier CA and $Ave_{dr2}$ of the classifier CB using the evaluation image. The update determination unit 23 controls whether the classifiers CA and CB, the learning image DB (determined) 100, and the learning image DB (before determination) 201 can be updated or creation of the classifiers CA and CB, the learning image DB (determined) 100, and the learning image DB (before determination) 201 by comparing a plurality of $Ave_{drX}$ obtained by the suitability evaluation unit 12.

That is, in a case in which $Ave_{dr2} > Ave_{dr1}$ is satisfied for all the images of the evaluation image, the classifier CB that is created based on the learning image DB (before determination) 201 collected, for example, from another facility or at another time, is more suitable for identification of the evaluation image than the classifier CA that is created based on the learning image DB (determined) 100 collected before. Therefore, the update determination unit 23 stores the classifier CB and the learning image DB (before determination) as a set with the evaluation image, separately from the classifier CA and the learning image DB (determined).

In the case in which $Ave_{dr2} \leq Ave_{dr1}$, the classifier CA that is created based on the learning image DB (determined) collected before is more suitable for the identification of the evaluation image than the classifier CB that is created based on the learning image DB (before determination) collected, for example, from another facility or at another time. Therefore, the update determination unit 23 stores the classifier CA and the learning image DB (determined) as a set with the evaluation image.

<Hardware Configuration of Machine Learning Device>

A configuration example of the hardware of the machine learning device 1 according to the present embodiment has the same configuration as that in FIG. 2. Unlike the machine learning device 1 according to the first embodiment, the memory 202 is provided with the update determination unit 23.

The auxiliary storage device 203 of the machine learning device 1 stores: the calculation result $Ave_{drX}$ obtained by the suitability evaluation unit 12; the classifiers CA and CB, the learning image DB (determined) 100, the learning image DB (before determination) 201, and the evaluation image that are determined by the update determination unit 23; and the parameters in Equations 1, 2, and 3 generated by the learning unit 11.

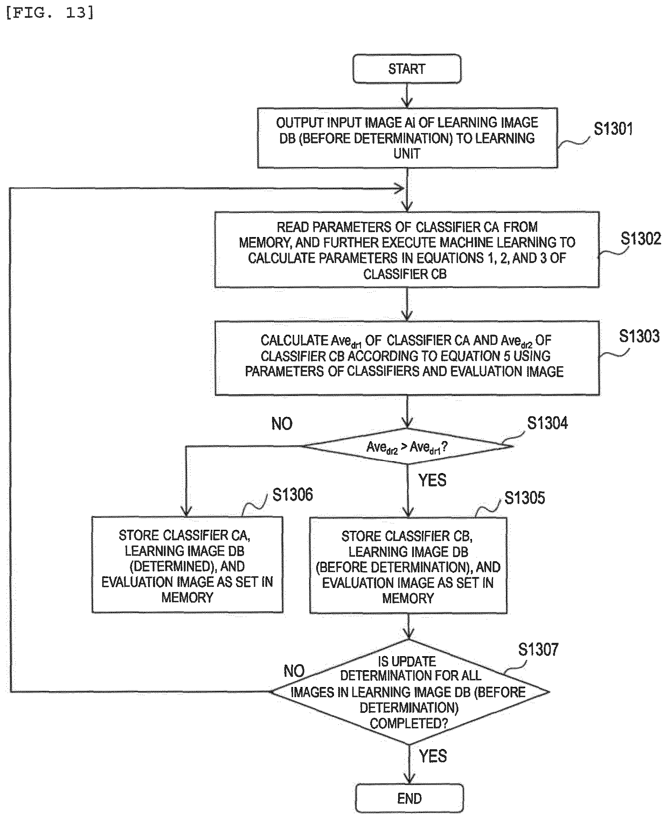

FIG. 13 is a flowchart illustrating an operation of the machine learning device 1 according to the present embodiment. Hereinafter, each processing unit (the input unit 10, the learning unit 11, and the like) is described as an operation subject. It may be interpreted that the CPU 201 is the operation subject, and the CPU 201 executes each processing unit as a program.

(i) Step 1301

The input unit 10 outputs the input image Ai of the learning image DB (before determination) 201 to the learning unit 11.

(ii) Step 1302

The learning unit 11 reads, from the memory 90, the parameters in Equations 1, 2, and 3 related to the classifier CA. The machine learning is executed using the learning image DB (before determination) 201, and the parameters in Equations 1, 2, and 3 related to the classifier CB is calculated.

(iii) Step 1303

The suitability evaluation unit 12 calculates $Ave_{dr1}$ of the classifier CA and $Ave_{dr2}$ of the classifier CB according to Equation 5 using the parameters of the classifiers CA and CB and the evaluation image.

(iv) Step 1304

The update determination unit 13 compares the calculated $Ave_{dr1}$ and $Ave_{dr2}$. In a case in which $Ave_{dr2} > Ave_{dr1}$, the update determination unit 13 proceeds the process to step 1305. On the other hand, in a case in which $Ave_{dr2} \leq Ave_{dr1}$, the update determination unit 13 proceeds the process to step 1306.

(v) Step 1305

The update determination unit 13 stores the classifier CB, the learning image DB (before determination) 201, the evaluation image, and the calculation results ($Ave_{dr2}$ and $Ave_{dr1}$) as a set in the memory 90 (equivalent to the auxiliary storage device 203).

(vi) Step 1306

The update determination unit 13 stores the classifier CA, the learning image DB (determined) 100, the evaluation image, and the calculation results ($Ave_{dr2}$ and $Ave_{dr1}$) as a set in the memory 90 (equivalent to the auxiliary storage device 203) .

(vii) Step 1307

The update determination unit 13 checks whether an update determination for all the images in the learning image DB (before determination) 201 is completed, and ends the process if the update determination is completed. On the other hand, if the update determination is not completed, the update determination unit 13 returns the process to step 1302 and repeats step 1302 to step 1306.

In the second embodiment, the machine learning is executed using the images in the plurality of learning image databases to create a plurality of classifiers, and the plurality of created classifiers are further evaluated to obtain evaluation results. In the second embodiment, the learning image database and the classifier that are to be used are determined by determining a plurality of evaluation results and controlling whether the classifier and the learning image database can be updated or creation of the classifier and the learning image database. Accordingly, it is possible to obtain, for each facility, the classifier capable of identifying the object (for example, a tissue or a cell) in the image with high accuracy and the learning image database.

The classifier, the learning image DB, the evaluation images, and the calculation results are set as a set to create data, so that performance of the classifier created according to data of another facility (a hospital or the like) can be determined by comparing the calculation results.

By changing the evaluation image into image data of any facility, it is possible to determine which classifier for a facility is used for the image of the facility to identify the object in the image with high accuracy.

The machine learning device 1 may hold a plurality of learning image DBs (determined) 100, and execute the above-mentioned process between each of the plurality of learning image DBs (determined) 100 and the learning image DB (before determination) 201. Accordingly, a more appropriate learning image DB and a more appropriate classifier can be obtained.

(3) Third Embodiment

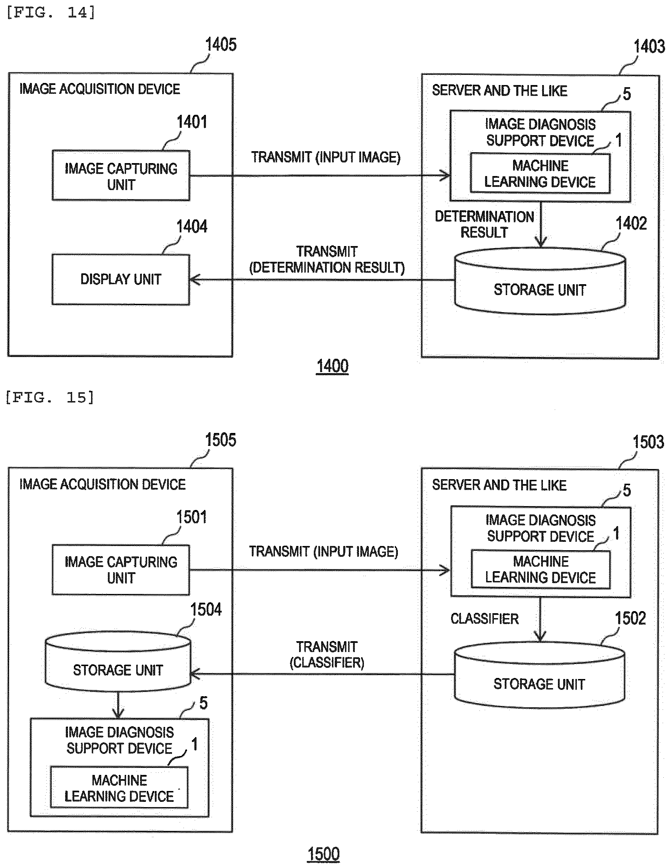

FIG. 14 is a functional block diagram illustrating a configuration of a remote diagnosis support system 1400 according to a third embodiment. The remote diagnosis support system 1400 includes a server (computer) 1403 and an image acquisition device 1405.

The image acquisition device 1405 is, for example, a device such as a virtual slide device or a personal computer equipped with a camera, and includes an image capturing unit 1401 that captures a new image and a display unit 1404 that displays a determination result transmitted from the server 1403. Although not illustrated, the image acquisition device 1405 includes a communication device that transmits image data to the server 1403 and receives data transmitted from the server 1403.

The server 1403 includes the image diagnosis support device 5 that executes, using the machine learning device 1 according to the first embodiment or the second embodiment, an image process on the image data transmitted from the image acquisition device 1405, and a storage unit 1402 that stores an identification result output from the image diagnosis support device 5. Although not illustrated, the server 1403 includes a communication device that receives the image data transmitted from the image acquisition device 1405 and transmits determination result data to the image acquisition device 1405.

The image diagnosis support device 5 identifies, for objects (for example, a tissue or a cell) in the image generated by the image capturing unit 1401, the presence or absence of an object (for example, an abnormal tissue or an abnormal cell (for example, a cancer)) to be detected using the classifier (the current classifier) obtained by the machine learning device 1. The display unit 1404 displays the identification result transmitted from the server 1403 on a display device screen of the image acquisition device 1405.

As the image acquisition device 1405, a regenerative medical device including an image capturing unit, a culture device for iPS cells, an MRI, an ultrasonic image capturing device, or the like may be used.

As described above, according to the third embodiment, it is possible to provide a remote diagnosis support system. Specifically, the remote diagnosis support system accurately classifies whether an object (for example, a tissue or a cell) in an image transmitted from a facility or the like at a different location is an object (an abnormal tissue, an abnormal cell, or the like) to be detected using the parameters of the classifiers obtained by the machine learning device 1. Further, a classification result is transmitted to the above-described facility or the like at a different location, and the classification result is displayed on the display unit of the image acquisition device in the facility or the like.

(4) Fourth Embodiment

FIG. 15 is a functional block diagram showing a configuration of a network contract service providing system 1500 according to a fourth embodiment. The network contract service providing system 1500 includes a server (computer) 1503 and an image acquisition device 1505.

The image acquisition device 1505 is, for example, a device such as a virtual slide device or a personal computer equipped with a camera. The image acquisition device 1505 includes an image capturing unit 1501 that captures a new image, a storage unit 1504 that stores a classifier (a current classifier) transmitted from the server 1503, and the image diagnosis support device 5.

The image diagnosis support device 5 reads the classifier transmitted from the server 1503, and determines whether an object (for example, a tissue or a cell) in an image newly captured by the image capturing unit 1501 is an object (for example, an abnormal tissue or an abnormal cell) to be detected using the classifier obtained by the machine learning device 1 according to the first embodiment or the second embodiment.

Although not illustrated, the image acquisition device 1505 includes a communication device that transmits image data to the server 1503 and receives data transmitted from the server 1503.

The server 1503 includes the image diagnosis support device 5 and a storage unit 1502 that stores a classifier output from the machine learning device 1 of the image diagnosis support device 5. The image diagnosis support device 5 creates a classifier from the machine learning device 1 according to the first embodiment or the second embodiment on the image data transmitted from the image acquisition device 1505, and further executes an identification process using the created classifier.

Although not illustrated, the server 1503 includes a communication device that receives image data transmitted from the image acquisition device 1505 and transmits a classifier to the image acquisition device 1505.

The machine learning device 1 in the image diagnosis support device 5 executes the machine learning so as to determine an object (for example, a tissue or a cell) in an image captured by the image capturing unit 1501 as an object (for example, a normal tissue or a normal cell is a normal tissue or a normal cell, and an abnormal tissue or an abnormal cell is an abnormal tissue or an abnormal cell) to be detected, and creates a classifier. The classifier calculates a feature amount of an object (for example, a tissue or a cell) in an image of a facility or the like at a different location. The storage unit 1504 stores the classifier transmitted from the server 1503.

The image diagnosis support device 5 in the image acquisition device 1505 reads the classifier from the storage unit 1504, classifies whether the object (for example, a tissue or a cell) in the image newly captured by the image capturing unit 1501 of the image acquisition device 1505 is an object (for example, an abnormal tissue or an abnormal cell) to be detected using the classifier, and displays the classification result on a display screen of the output device (display device) 204 of the image diagnosis support device 5.

As the image acquisition device 1505, the regenerative medical device including the image capturing unit, the culture device for iPS cells, the MRI, the ultrasonic image capturing device, or the like may be used.

As described above, according to the fourth embodiment, it is possible to provide a network contract service providing system. Specifically, the network contract service providing system executes the machine learning so as to classify an object (for example, a tissue or a cell) in an image transmitted from a facility or the like at a different location into an object (for example, a normal tissue or a normal cell is a normal tissue or a normal cell, and an abnormal tissue or an abnormal cell is an abnormal tissue or an abnormal cell) to be detected, and creates a classifier. The network contract service providing system transmits the classifier to the facility or the like at a different location, and reads the classifier by the image acquisition device in the facility or the like. The classifier in the image acquisition device classifies whether an object (for example, a tissue or a cell) in a new image is an object (for example, an abnormal tissue or an abnormal cell) to be detected.

The embodiments described above can be modified as follows. The learning unit 11 obtains a feature amount using a filter by executing the machine learning, and other feature amounts such as HOG may be used. The learning unit 11 may use a square error, a Hinge loss, or the like instead of the negative log likelihood as the loss function. The learning unit 11 may generate the classifier according to any method different from the method according to the above-mentioned embodiment.

The updating or generation of the classifier and the learning image database has been described in the above-mentioned embodiment. By changing the number of dimensions of input data to Equations 1 to 3 from two dimensions to another dimension, the updating or generation of the classifier and the learning database according to the above-mentioned embodiment can also be applied to a data sample different from an image, for example, a voice data sample, a sensor data sample, and a text data sample.

The invention can also be implemented by a program code of software for implementing the functions according to the embodiments. In this case, a storage medium in which the program code is recorded is provided to a system or a device, and a computer (or a CPU or an MPU) of the system or the device reads the program code stored in the storage medium. In this case, the program code itself read out from the storage medium implements the functions according to the above-described embodiment, and the program code itself and the storage medium storing the program code constitute the invention. Examples of the storage medium that supplies such a program code include a flexible disk, a CD-ROM, a DVD-ROM, a hard disk, an optical disk, a magneto-optical disk, a CD-R, a magnetic tape, a nonvolatile memory card, and a ROM.

An operating system (OS) or the like running on the computer may execute a part or all of an actual process based on an instruction of the program code, and the function according to the above-described embodiment may be implemented by the process. Further, after the program code read out from the storage medium is written in the memory on the computer, the CPU or the like of the computer may execute a part or all of the actual process based on the instruction of the program code, and the function according to the above-described embodiment may be implemented by the process.

Further, by distributing the program code of software for implementing the function according to the embodiment via a network, the program code may be stored in a storage unit such as a hard disk or a memory of a system or a device, or in a storage medium such as a CD-RW or a CD-R, and a computer (or a CPU or an MPU) of the system or the device may read out and execute the program code stored in the storage unit or the storage medium at the time of use.

Finally, processes and techniques described herein are not inherently related to any particular device, and can be implemented by any appropriate combination of components. Further, various types of devices for a general purpose can be used according to methods described herein. It may be beneficial to construct a dedicated device to execute the steps in the method described herein. Various inventions can be formed by appropriately combining the plurality of the components disclosed in the embodiments.

For example, some components may be deleted from all the components disclosed in the embodiments. Further, the components according to different embodiments may be appropriately combined. The invention is described in relation to specific examples, but these specific examples are for illustrative purposes only and not for purposes of limitation in all aspects. Those skilled in the art will recognize that there are numerous combinations of hardware, software, and firmware that are suitable for practicing the invention. For example, the described software can be implemented in a wide range of programs or script languages such as assembler, C/C++, perl, Shell, PHP, and Java (registered trademark).

Further, in the above-mentioned embodiments, control lines and information lines considered to be necessary for description are shown, and all of the control lines and information lines are not necessarily shown for the product. All configurations may be connected to one another.

In addition, other implementations according to the invention will be apparent to those skilled in the art in consideration of the specification and the embodiments of the invention disclosed herein. Various aspects and/or components according to the described embodiments can be used alone or in any combination.

## Claims

**1.** A machine learning device comprising:

a processor configured to process a data sample; and
a storage device configured to store a result of the process, wherein
the processor is configured to:

create a plurality of classifiers based on a plurality of learning databases, each of the plurality of learning databases storing a plurality of learning data samples;
create an evaluation result on identification performance of each of the plurality of classifiers; and
determine, based on the evaluation result, one learning database among the plurality of learning databases and a classifier to be generated based on the one learning database as a learning database and a classifier that are to be used.

**2.** The machine learning device according to claim 1, wherein

the data sample is an image, and
each of the plurality of learning databases is a learning image database.

**3.** The machine learning device according to claim 2, wherein

the plurality of learning databases include a first learning database and a second learning database,
the second learning database is configured to store images to be stored in the first learning database and new input images, and
the processor is configured to determine whether the first learning database is updatable according to the second learning database and whether a classifier to be generated based on the second learning database is usable.

**4.** The machine learning device according to claim 3, wherein
the processor is configured to determine, based on a comparison result between identification results of a first classifier generated based on the first learning database and a second classifier generated based on the second learning database, whether the first learning database is updatable according to the second learning database and whether a classifier to be generated based on the second learning database is usable.

**5.** The machine learning device according to claim 4, wherein
the processor is configured to:

determine whether to change an order of the new input images based on the comparison result between the identification result of the first classifier and the identification result of the second classifier;
create, when it is determined that the order is to be changed, a new second classifier based on the second learning database in which the order of the new input images is changed; and
determine, based on a comparison result between identification results of the first classifier and the new second classifier, whether the first learning database is updatable according to the second learning database and whether the classifier to be generated based on the second learning database is usable.

**6.** The machine learning device according to claim 3, wherein
in the first learning database and the second learning database, a balance of the number of images of identification types is adjusted.

**7.** The machine learning device according to claim 2, wherein

the plurality of learning databases include a first learning database and a second learning database,
the second learning database is configured to store a new input image different from an image stored in the first learning database, and
the processor is configured to determine to select and use one of the first learning database and the second learning database.

**8.** A remote diagnosis support system comprising:

an image acquisition device including an image capturing device configured to capture an image; and
a server including an image diagnosis support device provided with the machine learning device according to claim 2, wherein
the image diagnosis support device includes a current classifier that is generated and used by the machine learning device,
the image acquisition device is configured to transmit the image to the server,
the server is configured to process the received image by the image diagnosis support device, and transmit an image of an object identified by the current classifier and an identification result of the object to the image acquisition device, and
the image acquisition device is configured to display the received image of the object and the received identification result on a display device.

**9.** A network contract service providing system comprising:

an image acquisition device including an image capturing device configured to capture an image; and
a server including an image diagnosis support device provided with the machine learning device according to claim 2, wherein
the image diagnosis support device includes a current classifier that is generated and used by the machine learning device,
the server is configured to transmit the current classifier to the image acquisition device, and
the image acquisition device is configured to process the image captured by the image capturing device using the received current classifier, and display an image of an object identified by the current classifier and an identification result of the object on a display device.

**10.** An image diagnosis support device comprising:

a processor configured to process an image; and
a storage device configured to store a result of the process, wherein
the processor is configured to:

create a plurality of classifiers based on a plurality of learning image databases;
create an evaluation result on identification performance of each of the plurality of classifiers;
determine, based on the evaluation result, one learning image database among the plurality of learning image databases and a classifier to be generated based on the one learning image database as a learning image database and a classifier that are to be used; and
display an identification result of a new input image obtained by the classifier to be generated based on the one learning image database.

**11.** A machine learning method for a machine learning device to create a classifier, wherein

the machine learning device includes

a processor configured to process a data sample, and
a storage device configured to store a result of the process,

the machine learning method comprising:

creating, by the processor, a plurality of classifiers based on a plurality of learning databases, each of the plurality of learning databases storing a plurality of learning data samples;
creating, by the processor, an evaluation result on identification performance of each of the plurality of classifiers; and
determining, by the processor and based on the evaluation result, one learning database among the plurality of learning databases and a classifier to be generated based on the one learning database as a learning database and a classifier that are to be used.

**12.** The machine learning method according to claim 11, wherein

the data sample is an image, and
each of the plurality of learning databases is a learning image database.

**13.** The machine learning method according to claim 12, wherein

the plurality of learning databases include a first learning database and a second learning database, and
the second learning database is configured to store images to be stored in the first learning database and new input images, and
the machine learning method further comprising
determining, by the processor, whether the first learning database is updatable according to the second learning database and whether a classifier to be generated based on the second learning database is usable.

**14.** The machine learning method according to claim 13, wherein
the processor is configured to determine, based on a comparison result between identification results of a first classifier generated based on the first learning database and a second classifier generated based on the second learning database, whether the first learning database is updatable according to the second learning database and whether a classifier to be generated based on the second learning database is usable.

**15.** An image diagnosis support method using an image diagnosis support device, wherein

the image diagnosis support device includes

a processor configured to process an image, and
a storage device configured to store a result of the process,

the image diagnosis support method comprising:

creating, by the processor, a plurality of classifiers based on a plurality of learning image databases;
creating, by the processor, an evaluation result on identification performance of each of the plurality of classifiers;
determining, by the processor and based on the evaluation result, one learning image database among the plurality of learning image databases and a classifier to be generated based on the one learning image database as a learning image database and a classifier that are to be used; and
displaying, by the processor, an identification result of a new input image obtained by the classifier to be generated based on the one learning image database.

[FIG. 1]

1
IMAGE DATA (ex. MOVING IMAGE DATA)
MACHINE LEARNING DEVICE
CONTROL UNIT
91
INPUT UNIT
10
CONNECTED TO EACH ELEMENT
101
LEARNING IMAGE DB (DETERMINED + BEFORE DETERMINATION)
100
LEARNING IMAGE DB (DETERMINED)
LEARNING UNIT
11
102
SUITABILITY EVALUATION UNIT
12
EVALUATION IMAGE
UPDATE DETERMINATION UNIT
13
90
MEMORY
DRAWING UNIT
14
IDENTIFICATION RESULT

[FIG. 2A]

1
207
202
MEMORY
10
13(23)
201
CPU
INPUT UNIT
UPDATE DETERMINATION UNIT
204
11
14
OUTPUT DEVICE
LEARNING UNIT
DRAWING UNIT
205
12
INPUT DEVICE
SUITABILITY EVALUATION UNIT
206
203
COMMUNICATION DEVICE
AUXILIARY STORAGE DEVICE

[FIG. 2B]

LEARNING UNIT 11
111
112
113
FEATURE EXTRACTION UNIT
LOCAL IDENTIFICATION UNIT
OVERALL IDENTIFICATION UNIT

[FIG. 3]

FEA
FEATURE AMOUNT FAi
INPUT IMAGE
Ai
FEATURE EXTRACTOR
CNN (FIRST LAYER)
CNN (N-th LAYER)

[FIG. 4]

42 FILTER
41
TARGET IMAGE
(ex. PATHOLOGICAL
TISSUE IMAGE)

[FIG. 5]

FEATURE AMOUNT FAi
FILTER W
NONLINEAR
FUNCTION
NF
LOCAL
IDENTIFICATION
VALUE
LS
Y
X
m
fY
fX
C

[FIG. 6]

LS
BS
LOCAL IDENTIFICATION VALUE
BASIC IDENTIFICATION VALUE
TARGET OBJECT
OTHERS
DETERMINATION
S1

[FIG. 7]

INPUT IMAGE
Ai
FEA
LS
FEATURE EXTRACTOR
LOCAL IDENTIFICATION VALUE
BS
BASIC IDENTIFICATION VALUE
TARGET OBJECT
OTHERS
DETERMINATION
S1

[FIG. 8]

81
INPUT IMAGE
TUMOR
83
DETERMINATION RESULT OF LESION LIKELIHOOD
82
PORTION SUSPECTED AS ABNORMAL TISSUE OR ABNORMAL CELL

[FIG. 9]

START
OUTPUT LEARNING INPUT IMAGE TO LEARNING UNIT
S901
ACCORDING TO EQUATION 1, OBTAIN FEATURE AMOUNT FAi OF OBJECT USING FILTER, CREATE FEATURE EXTRACTOR FEA, AND CALCULATE FILTER COEFFICIENT wj AND OFFSET VALUES bi
S902
ACCORDING TO EQUATION 2, CALCULATE LOCAL IDENTIFICATION VALUE LS, COEFFICIENT OF FILTER W, AND OFFSET VALUE B USING FEATURE AMOUNT FAi AND FILTER
S903
ACCORDING TO EQUATION 3, CALCULATE COEFFICIENT OF FILTER W AND OFFSET VALUE B USING LOCAL IDENTIFICATION VALUE LS
S904
STORE RESPECTIVE PARAMETERS (FILTER COEFFICIENT wj, COEFFICIENT OF FILTER W, OFFSET VALUES bi AND B) IN EQUATIONS 1, 2, AND 3 IN MEMORY
S905
END

[FIG. 10]

START
OUTPUT INPUT IMAGE Ai OF LEARNING IMAGE DB (DETERMINED + BEFORE DETERMINATION) TO LEARNING UNIT
S1001
READ PARAMETERS OF CLASSIFIER CA FROM MEMORY, AND FURTHER EXECUTE MACHINE LEARNING TO CALCULATE PARAMETERS IN EQUATIONS 1, 2, AND 3 OF CLASSIFIER CB
S1002
CALCULATE $Ave_{dr1}$ OF CLASSIFIER CA AND $Ave_{dr2}$ OF CLASSIFIER CB ACCORDING TO EQUATION 5 USING PARAMETERS OF CLASSIFIERS AND EVALUATION IMAGE
S1003
$Ave_{dr2} > Ave_{dr1}$?
S1004
NO
YES
$Ave_{dr2}$ > THRESHOLD TH1?
S1006
NO
YES
$Ave_{dr2}$ > THRESHOLD TH1?
S1005
NO
YES
S1009
S1008
S1007
REPLACE CORRECT LABEL OF LEARNING IMAGE DB (BEFORE DETERMINATION)
CHANGE ORDER OF IMAGES IN LEARNING IMAGE DB (DETERMINED + BEFORE DETERMINATION)
UPDATE CLASSIFIER CA AND LEARNING IMAGE DB (DETERMINED)
IS UPDATE DETERMINATION FOR ALL IMAGES IN LEARNING IMAGE DB (DETERMINED + BEFORE DETERMINATION) COMPLETED?
S1010
NO
YES
STORE INFORMATION OF CLASSIFIER CA IN MEMORY
S1011
END

[FIG. 11]

・UPDATE STATUS
Avedr1
0.71
Avedr2
0.81
Mdr1
0.80
Mdr2
0.82
NUMBER OF UPDATES
10
Avedr1
NUMBER OF UPDATES


[FIG. 12]

IMAGE DATA (ex. MOVING IMAGE DATA)
1
CONTROL UNIT
91
CONNECTED TO EACH ELEMENT
INPUT UNIT
10
201
LEARNING IMAGE DB (BEFORE DETERMINATION)
100
LEARNING IMAGE DB (DETERMINED)
LEARNING UNIT
11
12
102
SUITABILITY EVALUATION UNIT
EVALUATION IMAGE
UPDATE DETERMINATION UNIT
23
90
MEMORY
DRAWING UNIT
14
IDENTIFICATION RESULT

[FIG. 13]

START
OUTPUT INPUT IMAGE AI OF LEARNING IMAGE DB (BEFORE DETERMINATION) TO LEARNING UNIT
S1301
READ PARAMETERS OF CLASSIFIER CA FROM MEMORY, AND FURTHER EXECUTE MACHINE LEARNING TO CALCULATE PARAMETERS IN EQUATIONS 1, 2, AND 3 OF CLASSIFIER CB
S1302
CALCULATE $Ave_{dr1}$ OF CLASSIFIER CA AND $Ave_{dr2}$ OF CLASSIFIER CB ACCORDING TO EQUATION 5 USING PARAMETERS OF CLASSIFIERS AND EVALUATION IMAGE
S1303
NO
S1304
$Ave_{dr2} > Ave_{dr1}$?
YES
S1306
S1305
STORE CLASSIFIER CA, LEARNING IMAGE DB (DETERMINED), AND EVALUATION IMAGE AS SET IN MEMORY
STORE CLASSIFIER CB, LEARNING IMAGE DB (BEFORE DETERMINATION), AND EVALUATION IMAGE AS SET IN MEMORY
S1307
NO
IS UPDATE DETERMINATION FOR ALL IMAGES IN LEARNING IMAGE DB (BEFORE DETERMINATION) COMPLETED?
YES
END

[FIG. 14]

1405
IMAGE ACQUISITION DEVICE
1401
IMAGE CAPTURING UNIT
1404
DISPLAY UNIT
TRANSMIT (INPUT IMAGE)
TRANSMIT (DETERMINATION RESULT)
1403
SERVER AND THE LIKE
5
IMAGE DIAGNOSIS SUPPORT DEVICE
1
MACHINE LEARNING DEVICE
DETERMINATION RESULT
1402
STORAGE UNIT
1400

[FIG. 15]

1505
IMAGE ACQUISITION DEVICE
1501
IMAGE CAPTURING UNIT
1504
STORAGE UNIT
5
IMAGE DIAGNOSIS SUPPORT DEVICE
1
MACHINE LEARNING DEVICE
TRANSMIT (INPUT IMAGE)
TRANSMIT (CLASSIFIER)
1503
SERVER AND THE LIKE
5
IMAGE DIAGNOSIS SUPPORT DEVICE
1
MACHINE LEARNING DEVICE
CLASSIFIER
1502
STORAGE UNIT
1500

## INTERNATIONAL SEARCH REPORT

International application No.
PCT/JP2020/036985

A. CLASSIFICATION OF SUBJECT MATTER
Int.Cl. A61B8/00(2006.01)i, G06T7/00(2017.01)i, G06N20/00(2019.01)i, A61B5/055(2006.01)i
FI: G06T7/00350B, G06T7/00614, G06N20/00, A61B5/055380, A61B8/00
According to International Patent Classification (IPC) or to both national classification and IPC

B. FIELDS SEARCHED

Minimum documentation searched (classification system followed by classification symbols)
Int.Cl. A61B8/00, G06T7/00, A61B5/055, G06N20/00

Documentation searched other than minimum documentation to the extent that such documents are included in the fields searched
Published examined utility model applications of Japan 1922-1996
Published unexamined utility model applications of Japan 1971-2020
Registered utility model specifications of Japan 1996-2020
Published registered utility model applications of Japan 1994-2020

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2019-109553 A (KONICA MINOLTA, INC.) 04 July 2019 (2019-07-04), paragraphs [0012]-[0084] | 1-4, 6, 7, 10-15 |
| Y | | 8, 9 |
| A | entire text | 5 |
| Y | JP 2018-180794 A (HITACHI HIGH-TECHNOLOGIES CORP.) 15 November 2018 (2018-11-15), paragraphs [0102]-[0116] | 8, 9 |

☐ Further documents are listed in the continuation of Box C.
☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
26 October 2020

Date of mailing of the international search report
10 November 2020

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

**INTERNATIONAL SEARCH REPORT**
Information on patent family members

International application No.
PCT/JP2020/036985

| | | |
|---|---|---|
| JP 2019-109553 A | 04 July 2019 | US 2019/0189278 A1<br>paragraphs [0031]-[0247] |
| JP 2018-180794 A | 15 November 2018 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019181165 A
- JP 2016143351 A